(19) Europäisches Patentamt / European Patent Office / Office européen des brevets

(11) **EP 4 576 101 A1**

(12) **EUROPEAN PATENT APPLICATION**
published in accordance with Art. 153(4) EPC

(43) Date of publication:
**25.06.2025 Bulletin 2025/26**

(21) Application number: **23854441.5**

(22) Date of filing: **15.08.2023**

(51) International Patent Classification (IPC):
**G16B 20/00** (2019.01)

(52) Cooperative Patent Classification (CPC):
**G16B 20/00; G16B 30/00**

(86) International application number:
**PCT/CN2023/113130**

(87) International publication number:
**WO 2024/037540 (22.02.2024 Gazette 2024/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.08.2022 CN 202210982600**

(71) Applicant: **Zhejiang Huode Bioengineering Company Limited**
**Hangzhou, Zhejiang 310018 (CN)**

(72) Inventors:
• **FAN, Jing**
  **Hangzhou, Zhejiang 310018 (CN)**
• **CAI, Zhengwen**
  **Hangzhou, Zhejiang 310018 (CN)**
• **WANG, Anxin**
  **Hangzhou, Zhejiang 310018 (CN)**

(74) Representative: **Lewis Silkin LLP**
**Arbor**
**255 Blackfriars Road**
**London SE1 9AX (GB)**

(54) **BIOLOGICAL SAMPLE CELL COMPOSITION DETECTION METHOD AND APPARATUS, DEVICE, AND STORAGE MEDIUM**

(57)    The present application discloses a biological sample cell composition detection method and apparatus, an electronic device, and a readable storage medium, which are applied to the technical field of biomedicine. The method comprises: performing single-cell transcriptome sequencing on a biological sample to be detected to obtain a single-cell sequencing result, generating a cell gene expression matrix by analyzing the single-cell sequencing result, and performing single-cell bioinformatics analysis on the cell gene expression matrix to determine cell types comprised in said biological sample. The present application can accurately and quantitative detect the cell composition of a biological sample in one step with low cost and high throughput

FIG.1

# EP 4 576 101 A1

## Description

### CROSS-REFERENCE TO RELATED APPLICATIONS

This application is a National Stage Application and claims priority under 35 U.S.C. § 371 to Patent Cooperation Treaty application PCT/CN2023/113130, filed August 15, 2023, which claims the benefit of Chinese application CN202210982600.7, filed August 16, 2022. Priority is claimed to these applications and the disclosures of these prior applications are considered part of the disclosure of this application and to the extent allowed the entire contents of the aforementioned applications are incorporated herein.

### Technical Field

The present application relates to the field of biomedical technology, and in particular to a method, an apparatus, an electronic device and a readable storage medium for detecting the cell composition of a biological sample.

### Background

With the development of regenerative medicine technology, stem cells have a very advantageous application prospect in the construction of organ-like disease models, research on the mechanism of new drugs, and diseases that do not yet have effective drugs and require cell transplantation. Stem cells are cells with the ability of self-renewal and differentiation, among which embryonic stem cells and induced pluripotent stem cells are collectively known as pluripotent stem cells because of their potential to differentiate into all cells in the human body. Based on the application of stem cell-derived cell products in the above fields, it is necessary to carry out sensitive and accurate cell type confirmation to confirm that the cell products are mainly composed of the expected differentiated cells, with stable and controllable cell composition, batch-to-batch homogeneity, and reproducibility of the overall function, especially in the treatment of diseases, to ensure that there is no deviation in the direction of cell differentiation, and that there is no unintended differentiated cell that is harmful to human beings, so as to ensure the safety and effectiveness of the drugs.

Currently, the commonly used methods for cell type identification include qPCR (Quantitative Real-time PCR (Polymerase Chain Reaction)), dPCR (Digital PCR), FISH

(Fluorescence in situ hybridization technique based on nucleic acid molecular hybridization) and IF (Immunofluorescence technique, antibody-based immunofluorescence staining), FC (Flow Cytometry). Among them, qPCR and dPCR identify cell types by targeting and amplifying functional cell-specific genes to analyze expression levels. FISH uses fluorescently labeled specific nucleic acid probes to hybridize with corresponding target DNA or RNA molecules within cells, allowing for the identification of cell types by observing fluorescent signals under a fluorescence microscope or confocal laser scanner. IF, on the other hand, uses fluorescently labeled specific antibodies that, through antigen-antibody reactions, localize cell antigen substances, enabling cell type identification by observing fluorescent signals. FC analyzes the physical, chemical, and functional properties of cells, distinguishing cell types by specifically expressed proteins to achieve qualitative or quantitative cell type detection. All the above methods require the design or production, or procurement of specific primers or specific antibodies based on genes or molecular markers specific to a known cell type before a known cell type can be detected. It is difficult to perform the detection of an unknown cell type by the above methods when the cell type cannot be predicted in advance or when very specific molecular markers or specific antibodies are not available for certain types of cells. In addition, all of them are difficult to analyze multiple cell types on the same sample at the same time, and the information obtained is very limited, making high-throughput cell identification and quantification impossible. Moreover, since the detection of cell types is based on the specific expression of several genes or protein targets in functional cells, due to the small number of targets detected, the heterogeneity of target expression in different states of cells or nonspecific expression, the results may cause certain false positives or false negatives, and the data from the assay cannot be explored and analyzed in depth.

In summary, the relevant technologies, whether protein-based or nucleic acid-based target detection methods, have the disadvantages of being difficult to analyze unknown samples, or high cost and low throughput of detection by matrix screening, as well as being highly affected by limited molecular markers and the specificity of antibodies and probes.

In view of this, how to achieve compositional detection of sample cells in one step with low cost, high throughput, and accurate quantification is a technical problem to be solved by the technicians in the field to which it belongs.

### Summary

The present application provides a method, apparatus, electronic device, and readable storage medium for detecting cell composition of a biological sample, which enables low-cost, high-throughput, accurate and quantitative one-step detection of cell composition of a biological sample.

To address the above technical problems, embodiments of the present invention provide the following technical solutions:

An embodiment of the present invention provides, on the one hand, a method for detecting the cell composition of a biological sample, comprises:

Performing single-cell transcriptome sequencing on the biological sample to be detected to obtain single-cell sequencing results.

Generating a cell gene expression matrix by analyzing the said single-cell sequencing results.

Determining the cell types contained in said biological sample to be detected by performing single-cell bioinformatics analysis of said cell gene expression matrix.

Optionally, said analyzing said cell gene expression matrix by performing single-cell bioinformatics analysis, further comprises:

Mapping functional cell gene expression matrix information to said cell gene expression matrix for initial cell annotation.

Optionally, said mapping of functional cell gene expression matrix information to said cell gene expression matrix is followed by:

Generates and displays functional cell target gene expression and cell expression ratio maps in response to functional cell target gene expression clustering mapping commands.

In response to an annotation confirmation result input command, an annotation confirmation message containing the cell type to which each cell taxon belongs is generated.

Optionally, said analyzing said cell gene expression matrix by performing single-cell bioinformatics analysis of said cell, further comprises:

Generate gene splicing data by analyzing said single cell sequencing results.

Based on said gene splicing data, a trajectory analysis of the annotated cell taxa is performed to confirm whether the cell annotation taxa fit the biological developmental trajectory.

Optionally, said determining the cell types contained in said biological sample to be detected is followed by:

Calculating cell composition ratios and generating a cell composition ratio result for said biological sample to be detected.

Optionally, said determining the type of cell contained in said biological sample to be detected by performing a single-cell bioinformatics analysis of said cell gene expression matrix comprises:

Performing single-cell transcriptional data analysis of said cell gene expression matrix in an interactive computing environment to obtain the type of cell contained in said biological sample to be detected.

Optionally, said determining the cell types contained in said biological sample to be detected by performing a single-cell bioinformatics analysis of said cell gene expression matrix comprises:

A cell gene calculation relational formula is invoked to count the proportion of cell mitochondrial genes, the total number of genes detected by the cell, the total number of gene fragments detected by the cell and the total number of fragments detected by the gene and the total number of cells measured in said cell gene expression matrix.

In response to the filtering parameter setting command, the cell filtering relational formula, and the gene filtering relational formula are invoked respectively to filter out the genes and cells whose detection quality does not satisfy the preset quality conditions and obtain the target cell gene data.

In response to a data normalization processing instruction, data normalization processing is performed on said target cell gene data, and dimensionality reduction processing is performed on the normalized data.

In response to the cell clustering processing command, cell clustering is performed on the dimensionality reduction processed data to obtain the cell subcluster information.

Optionally, said obtaining the target cell genetic data further comprises:

A cell cycle assessment relational formula is invoked to determine the cell cycle of each cell in said target cell genetic data.

Optionally, said data normalization of said cell gene expression matrix in response to a data normalization processing instruction comprises:

A normalization relation is invoked to normalize said cell gene expression matrix.

Call the logarithmic conversion relational formula to perform logarithmic conversion of normalized processing data.

Call the abnormal gene removal relation formula to remove abnormally high expressed genes from the log-transformed data.

Optionally, said biological sample to be detected comprises a plurality of batches of biological samples, and said single cell sequencing results comprise a plurality of sets of single cell sequencing results carrying the batch information; said determining the type of cells comprised in said biological sample to be detected followed by:

Inter-batch cell composition ratio stability results are generated by analyzing cell composition ratio data for each batch of biological samples.

Optionally, said biological sample to be detected is sampled at a plurality of time points of the same biological sample, and said single-cell sequencing results comprise single-cell sequencing results at a plurality of time points of the same biological sample; said determining the type of cells contained in said biological sample to be detected after further comprises:

For each time point of the bio-sample, obtain data on the proportion of cell composition determined for the bio-sample at the current time.

Information on changes in cell composition proportions is generated by time-series analysis of cell composition proportions data from biological samples at different times.

Embodiments of the present invention provide, on the other hand, a biological sample cell composition detection device comprises:

Sequencing module for performing single-cell transcriptome sequencing of a biological sample to be detected and obtaining single-cell sequencing results.

A data analysis module for generating a cell gene expression matrix by analyzing said single cell sequencing results.

A cell type determination module for determining a cell type contained in said biological sample to be detected by performing a single-cell bioinformatics analysis of said cell gene expression matrix.

Embodiments of the present invention also provide an electronic device comprising a processor, said processor being used to implement the steps of a method of detecting the cell composition of a biological sample as described in the previous item when executing a computer program stored in memory.

Embodiments of the present invention finally provide a readable storage medium, said readable storage medium having stored there on a computer program, said computer program being executed by a processor to implement the steps of a method for detecting the cell composition of a biological sample as described in the previous item.

The advantage of the technical solution provided by the present application is that the biological information analysis based on the single-cell sequencing results of the sample to be detected can deeply explore the cell characteristic information, and can present the gene expression and transcription level of each cell in the state of detection at the single-cell level, which can effectively improve the stability of the detection of the entire biological sample, help to avoid false-positive or false-negative judgement errors, and effectively improve the accuracy of the detection. In addition, the whole analysis is carried out through a modular procedure, which does not require high experience of operators, can effectively reduce the cost of cell composition detecting, and meets the requirements for the stability and reproducibility of the detect. At the same time, the gradual accumulation of detect data can be combined with the iterative development of technology for repeated mining analysis and utilization, which can further enhance the accuracy of the cell composition of biological samples.

In addition, embodiments of the present invention provide corresponding realization devices, electronic devices and readable storage media for the method of detecting the cell composition of a biological sample, which further makes the said method more practical, and the said devices, electronic devices and readable storage media have corresponding advantages.

It should be understood that the above general description and the detailed description that follows are merely exemplary and do not limit the present disclosure.

**Brief Description of Figures**

In order to more clearly illustrate the technical solutions of the embodiments of the present invention or related technologies, the following will briefly introduce the accompanying drawings that need to be used in the description of the embodiments or related technologies, and it is obvious that the accompanying drawings in the following description are only some embodiments of the present invention, and for the person of ordinary skill in the field, other accompanying drawings can be obtained according to these drawings without giving creative labor.

FIG.1 shows a schematic flow diagram of a method for detecting the cell composition of a biological sample.
FIG.2 shows a structural diagram of one specific embodiment of a cell composition detection device for biological sample.
FIG.3 shows a diagram of the structure of a specific embodiment of an electronic device.
FIG.4 shows a schematic flow diagram of another method of detecting cell composition of a biological sample.
FIG.5 shows a violin plot of the total number of gene detect performed on the sample cells before filtering.
FIG.6 shows a violin plot of the total number of gene fragments detected before filtering of the sample cell.
FIG.7 shows a plot of the mitochondrial gene detection ratio violin before sample cell filtration.
FIG.8 shows a plot of the total number of violins genetically detected after filtering of the sample cell.
FIG.9 shows a violin plot of the total number of gene fragments detected after filtering of the sample cell.
FIG.10 shows a plot of the mitochondrial gene detection ratio violin after sample cell filtering.

FIG.11 shows a plot of the data downscaling cell cycle distribution.

FIG.12 shows the data dimensionality reduction functional cell matrix mapping cell annotation map.

FIG.13 shows the functional cell matrix mapping annotated cell composition scale pie chart.

FIG.14 shows the data dimensionality reduction cell clustering map.

FIG.15 shows a cell clustering gene expression bubble map.

FIG.16 shows the data dimensionality reduction target gene expression correction cell annotation plot cell annotation plot.

FIG.17 shows a plot of the data degradation cell trajectory.

FIG.18 shows a pie chart of the proportion of biological developmental trajectories confirming the composition of cell annotations.

FIG.19 shows a pie chart of cell cycle ratios.

FIG.20 shows a scaled bar graph of cell cycle composition.

FIG.21 shows a scaled bar graph of the cell composition cycle.

FIG.22 shows the data downscaling based on SingleR software multi-cell annotation database cell annotation map.

FIG.23 shows a pie chart of the proportional composition of cell annotations based on the SingleR software multi-cell annotation database.

## Detailed Description

In order to enable those skilled in the art to better understand the solutions of the present invention, the following provides a further detailed description of the present invention in conjunction with the accompanying drawings and specific embodiments. It is evident that the described embodiments are merely a part of the embodiments of the present invention, rather than all the embodiments. All other embodiments obtained by those skilled in the art based on the embodiments of the present invention, without making any creative efforts, fall within the protection scope of the present invention.

The terms "comprising" and "having", and any variations thereof, in the specification and claims of the present application and in the accompanying drawings referred to above, are intended to cover non-exclusive inclusion. For example, a process, method, system, product or apparatus comprising a series of steps or units is not limited to the listed steps or units but may include steps or units that are not listed.

The term "a method of assaying the cell composition of a biological sample" in the specification and claims of the present application and the accompanying drawings described above refers to a method for determining the type of cells contained in a biological sample and/or for generating a proportional assay result for the cell composition of a biological sample.

Having described the technical solutions of the embodiments of the present invention, various non-limiting embodiments of the present application are described in detail below.

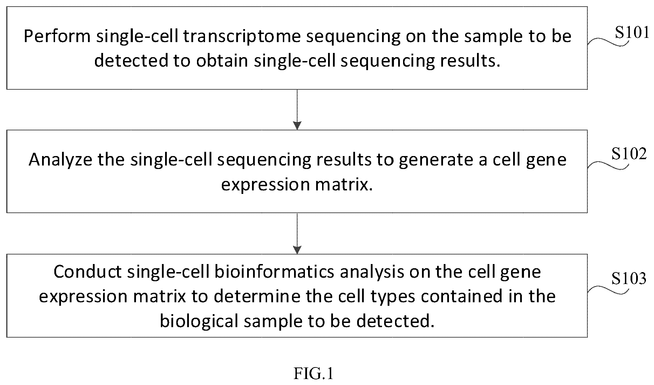

Referring first to FIG.1, FIG.1 shows a schematic flow diagram of a method for detecting cell composition of a biological sample provided by an embodiment of the present invention, which may comprise the following:

S101: The biological sample to be detected is subjected to single-cell transcriptome sequencing, and single-cell sequencing results are obtained.

In this embodiment, the sample to be detected may be a cell-dissociated sample or a tissue-dissociated sample, neither of which affects the realization of the present application. Single-cell sequencing results are obtained by extracting and amplifying the genome or transcriptome and analyzing it by high-throughput sequencing at the level of a single cell, and the single-cell sequencing results are used to represent the single-cell genetic information, or rather the gene structure and gene expression status unique to a single cell. This embodiment may use any kind of software that enables analysis and comparison of single cell transcriptome sequencing data to obtain a cell gene expression matrix, for example, Cell ranger on the 10X GENOMICS platform may be used, and software such as CeleScope software, STARsolo software, etc., which do not affect the implementation of the present application. Increasing the amount of single-cell sequencing data refers to the sequencing technology, i.e., the technology is capable of performing high depth, i.e., greater than 100G single-cell transcriptome sequencing of the biological samples to be detected in order to further improve the performance of the single-cell sequencing, wherein G is the size of the amount of sequencing data, and 1G represents one billion bases, e.g., one million cell samples or tissues can be used for dissociating them into a single cell, and then using the 10X For example, 1,000,000 cell samples or tissues can be dissociated into single cells and then sequenced using the 10X GENOMICS sequencing platform for 100G deep single-cell transcriptome sequencing.

S102: Generation of cell gene expression matrix by analyzing single-cell sequencing results.

In this step, a comparison index may first be constructed based on the single-cell sequencing results by referring to a human genome such as the GRCh38 version of the genome and a gene annotation information database such as the GENECODE database annotation file, wherein the comparison index is used in the process of sequence comparison, i.e., in the process of converting the sequence file into a cell gene expression matrix. Further the single cell sequencing data expression matrix may be obtained by genomic comparison and cell quantification of the single cell transcriptome data, and the cell gene expression matrix may be obtained after processing and filtering the single cell sequencing data expression matrix.

S103: Determine the cell types contained in the biological sample to be detected by performing a single-cell bioinformatics analysis of the cell gene expression matrix.

In this step, in order to improve the accuracy and detection efficiency of the cell composition detection of the biological sample, this step may execute this single-cell bioinformatics analysis process in an interactive computing environment, and by executing the entire data analysis process in an interactive computing environment, the parameters may be adjusted in a timely manner in accordance with the results of each step of the operation, so as to achieve the purpose of flexible processing. The single-cell bioinformatics analysis of the present embodiment refers to performing a series of data analysis operations on a cell gene expression matrix using a biological analysis means for the purpose of ultimately obtaining a cell type contained in the biological sample to be detected. The step may be performed by invoking an existing single-cell transcriptional data analysis function such as Scanpy to perform a single-cell transcriptional data analysis on the cell gene expression matrix in an interactive computing environment to obtain functional cell gene expression matrix information. Optionally, the cell gene expression matrix may be analyzed by Scanpy based on the Python jupyter notebook visual manipulation platform, or the cell gene expression matrix may be analyzed by Seurat software based on the R language platform.

In the technical solution provided by an embodiment of the present invention, the bioinformatic analysis based on the single-cell sequencing results of the sample to be detected can deeply excavate the cell characteristic information, and can present the gene expression and transcription level of each cell in the state at the time of detecting at the single-cell level, which can effectively improve the stability of the detecting of the entire biological sample, and is conducive to the avoidance of false positives or false negatives, which can effectively improve the accuracy of the detecting. In addition, the whole analysis is carried out through a modular procedure, which does not require high experience of operators, can effectively reduce the cost of cell composition detecting, and meets the requirements for the stability and reproducibility of the detect. At the same time, the gradual accumulation of detect data can be combined with the iterative development of technology for repeated mining analysis and utilization, which can further enhance the accuracy of the cell composition of biological samples.

In order to further improve the accuracy of the detection, and to avoid false positive or false negative judgement by the operator due to the use of a small number of targets, leading to inaccurate results of the final cell detection, based on the above embodiment, the present application may also use the functional cell gene expression matrix mapping for rapid identification prior to the step of determining the cell type contained in said biological sample to be detected in S103, i.e., to further improve the accuracy of the detection, and the application may also use the functional cell gene expression matrix mapping to further improve the accuracy of the detection, based on the above embodiment. Based on the above embodiment, after a single cell bioinformatics analysis is performed by performing a single cell bioinformatics analysis on said cell gene expression matrix, the functional cell gene expression matrix information may also be mapped to the cell gene expression matrix for preliminary cell type annotation. After obtaining the information related to the cell type annotation, the cell composition ratio may be counted based on the cell annotation information to generate the cell composition ratio detection result of the biological sample to be detected.

The single-cell transcriptome sequencing yields unlabeled data, in order to determine the cell type of the single-cell transcriptome sequencing data corresponding to the biological sample to be detected, the cell type can be determined by performing a similarity comparison with the existing data with annotations. In this step, the annotated functional cell gene expression matrix information may be mapped to the cell gene expression matrix corresponding to the original single-cell sequencing results for rapid identification of the cell type. The functional cell used in the functional cell gene expression matrix information may be any cell selected by a person skilled in the art for more practical application scenarios, and the functional cell is analyzed by single-cell transcriptome sequencing and single-cell sequencing results to obtain the functional cell gene expression matrix information. Therein, the functional cell gene expression matrix information may be mapped to the cell gene expression matrix using, for example, scGCN software, singleR software, etc., and of course, other types of annotation techniques may be used to realize the mapping of the functional cell gene expression matrix information to the cell gene expression matrix.

After the initial annotation of the cell types, based on the initial annotation results, a proportion of each type of cell comprising the biological sample to be detected is obtained by counting the proportion of each type of cell of the biological sample to be detected. The cell composition ratio detection result can be used to indicate the type of cells contained in the biological sample to be detected or directly for the cell composition ratio data of the biological sample to be detected, and the cell composition ratio detection result can be a document in any format, such as a word document, a PDF text, or an excel table, and, of course, can be exported as a picture, an audio-video, and so on, which do not affect the realization of the present application.

The above embodiment uses functional cell gene expression matrix mapping for rapid identification during bioinformatics analysis of single-cell sequencing data, and in order to further improve the accuracy of cell type detection, based on the above embodiment, the present embodiment may further comprise, after carrying out the step of the above embodiment of "mapping the functional cell gene expression matrix information to said cell gene expression matrix to carry out preliminary cell annotation", it may further include:

In response to the functional cell target gene expression clustering graph drawing instruction, generate and display the functional cell target gene expression amount and cell expression proportion graph, such as bubble graph, scatter graph, trajectory graph, heat map and so on. In response to the annotation confirmation result input command, generate annotation confirmation information containing the cell type to which each cell taxon belongs.

The present embodiment may employ any kind of drawing software to draw the target gene expression clustering map of the functional cells used in the above embodiment, and confirm the accuracy of the cell type annotation of the above embodiment based on the manual experience target gene expression clustering map, and through the human-computer interaction module, the cell types belonging to the cell taxa in the biological samples to be detected may be further inputted into the system, and the system generates an annotation confirmation message for the preliminary cell annotation based on the received information. cell annotation based on the information received, and the system generates an annotation confirmation message for the preliminary cell annotation.

The present embodiment, while using the functional cell gene expression matrix mapping for rapid identification, also combines multi-target gene cross-validation to further confirm the cell type, developmental state, and cell cycle characteristics, avoiding false-positive or false-negative judgements brought about by the use of a small number of targets in the above-described technology, and further enhancing the accuracy of the detection of the cell composition of the biological sample. Accordingly, based on the annotation confirmation information, the cell composition ratio detection results of the above embodiment are verified, and optionally, the cell composition ratio can be counted again based on the annotation confirmation information, and then the cell composition ratio detection results of the biological sample to be detected can be updated.

In order to achieve accurate qualitative and quantitative detection of the type of cells contained in the biological sample and quantitative detection of the proportion of cell composition of the biological sample to be detected, based on the above embodiments, it may further comprise:

Gene splice data is generated by analyzing said single cell sequencing results; based on the gene splice data, trajectory analysis is performed on the annotated cell taxa to confirm whether the cell annotated taxa are fitted to a biological developmental trajectory.

This embodiment may process the sequence comparison file using any of the RNA rate analysis techniques to obtain gene splice data. The gene splice data is used to analyze cell developmental trajectories. This step may use any kind of biological development trajectory analysis software to perform trajectory analysis on the annotated cell taxa, such as scvelo, monocle, CellRank, etc., which may be used to perform trajectory analysis on the biological samples to be detected, to obtain the validation results of the trajectory analysis of the biological samples to be detected, and to determine whether they are fitted to the biological development trajectories of the corresponding types of cell taxa.

Accordingly, in order to verify the accuracy of the cell composition ratio detection results of the above embodiment, the cell type annotation results may be further confirmed based on the target gene expression, i.e., the annotation confirmation information and the trajectory analysis verifies the cell type annotation results, the cell composition ratio is again statistically calculated, and finally, the cell composition ratio detection results of the generated biological samples to be detected are updated based on the current statistically calculated cell composition ratio.

This embodiment confirms whether the cell subpopulation is fitted to the biological developmental trajectory by trajectory analysis, and counts the percentage of each type of cell, to achieve accurate qualitative and quantitative detection of the cell type of the sample, and quantitative detection of the proportion of the cell composition of the biological sample to be detected.

Based on the above embodiment, to further improve the richness of the cell composition detection results and enhance the user experience, based on the above embodiment, the cell composition detection results of the biological sample to be detected in the present embodiment may include more data, and optionally, the present embodiment may also include the following:

According to the proportion information of each type of cell, call the plotting function to generate the cell type distribution pie chart and each type of cell cycle histogram; based on the cell type distribution pie chart and each type of cell cycle histogram, generate the cell composition proportion detection results.

In this embodiment, the plotting function may include a plotting function for generating a pie chart of the distribution of the cell types, and further includes a plotting function for generating a histogram of the distribution of the cell cycle of each type. Of course, the cell composition ratio detection results, in addition to the ratio of each type of cell of the biological sample to be detected, the rest of the information, such as the trajectory analysis validation results, the expression amount of the functional cell target genes and the cell expression ratio graph, the annotation confirmation information, the cell type distribution pie charts, and the various types of cell cycle histograms, may be flexibly selected according to the actual needs, which do not affect the realization of the present application.

Without limiting how the above embodiment performs the step "determining the type of cell contained in said biological sample to be detected by performing a single-cell bioinformatics analysis of the cell gene expression matrix", the present embodiment gives an optional embodiment, which may comprise the following:

A cell gene calculation relational formula is invoked to count the proportion of cell mitochondrial genes, the total number of genes detected by the cell, the total number of gene fragments detected by the cell and the total number of fragments detected by the gene and the total number of cells measured in said cell gene expression matrix.

In response to the filtering parameter setting command, the cell filtering relational formula, and the gene filtering relational formula are invoked respectively to filter out the genes and cells whose detection quality does not satisfy the preset quality conditions and obtain the target cell gene data.

Call the cell cycle assessment relational formula to determine the cell cycle of each cell in the target cell's genetic data.

Responding to data normalization commands, data normalization is performed on the target cell genetic data and dimensionality reduction is performed on the normalized data.

In response to the cell clustering processing command, cell clustering is performed on the dimensionality reduction processed data to obtain the cell subgroup information.

In this embodiment, any kind of clustering algorithm may be used to perform the cell clustering process, e.g., a clustering function may be used to perform the clustering directly, and the clustering function may, for example, be Leiden, Louvain, etc., and the present application does not make any limitation thereon.

The above embodiments do not qualify how the data normalization operation is to be carried out, and the present embodiments also give an optional embodiment of data normalization which may include the following:
Normalization of the cell gene expression matrix by calling the normalization relation.

Call the logarithmic conversion relational formula to perform logarithmic conversion of normalized processing data.

Call the abnormal gene removal relation formula to remove abnormally high expressed genes from the log-transformed data.

Based on the above embodiment, in order to further improve the richness of the cell type detection report and enhance the user experience, based on the above embodiment, the detection result report of the biological sample to be detected in the present embodiment further may include more data, and optionally, the cell composition ratio detection result may also include the target cell gene data, the downscaling processing data, the cell classification information, and the like, which may also be generated based on the biological developmental trajectory validation results, the target cell genetic data, the downscaling processing data, the cell classification information, and the proportion of each type of cell, the cell composition ratio detection results can also be generated.

In addition, in order to further explore the stability of the cell composition ratio of different batches of biological samples or the variation of the cell composition ratio of biological samples at different time points, based on the above embodiments, the present embodiments may also carry out an integrated analysis to verify the stability of the cells between batches, carry out a time-series analysis of the cells of the same batch of cells with different culture times, and monitor the variation of the cell composition ratio, and the present embodiments may comprise the following elements: The biological sample to be detected may comprise a plurality of batches of biological samples, and accordingly, the single-cell sequencing results comprise a plurality of sets of single-cell sequencing results carrying batch information; after generating a report of the detection results of said biological sample to be detected, generating a result of the stability of the inter-batch cell composition ratio by analyzing the cell composition ratio data of biological samples in each batch, so as to realize that by integrating the analysis of multiple batches of the detected samples, the stability of the inter-batch cell composition ratio can be verified. to verify the inter-batch cell composition ratio stability.

For the same biological sample to be detected, the sample may be acquired at multiple time points, and the single-cell sequencing results include the single-cell sequencing results of the same biological sample at multiple time points, and the biological sample acquired at each time point is processed in accordance with the method of the above embodiment, and the cell composition ratio data determined for the biological sample at the current moment is acquired, and by performing a time-series analysis of the cell composition ratio data at different times, the Generate information on the change of cell composition ratio by time series analysis of cell composition ratio data at different time. The time series analysis is carried out through multiple detecting of the same batch to monitor the change of cell composition ratio.

The present embodiment, by verifying the cell stability of multiple batches of cells, combined with the results of time-series analysis of cells at different culture times of the same biological sample, can further achieve the accuracy of the detection of the proportion of the cell composition of the biological sample to be detected, and can also be further explored to determine the stability of the cell composition of the biological sample or the changes in the cell development process.

It should be noted that there is no strict sequential order of execution between the steps in the present application, if they conform to a logical order, then these steps may be executed simultaneously or in a certain pre-determined order, and FIG.1 is only an illustrative way, and does not mean that it can only be such an order of execution.

Embodiments of the present invention also provide corresponding devices for the method of detecting the cell composition of a biological sample, further making the method more practical. Among other things, the device may be described from the perspective of a functional module and from the perspective of hardware, respectively. The biological sample cell composition detection device provided in the embodiments of the present invention is described below, and the biological sample cell composition detection device described hereinafter, and the biological sample cell composition detection method described hereinabove may be referred to each other correspondingly.

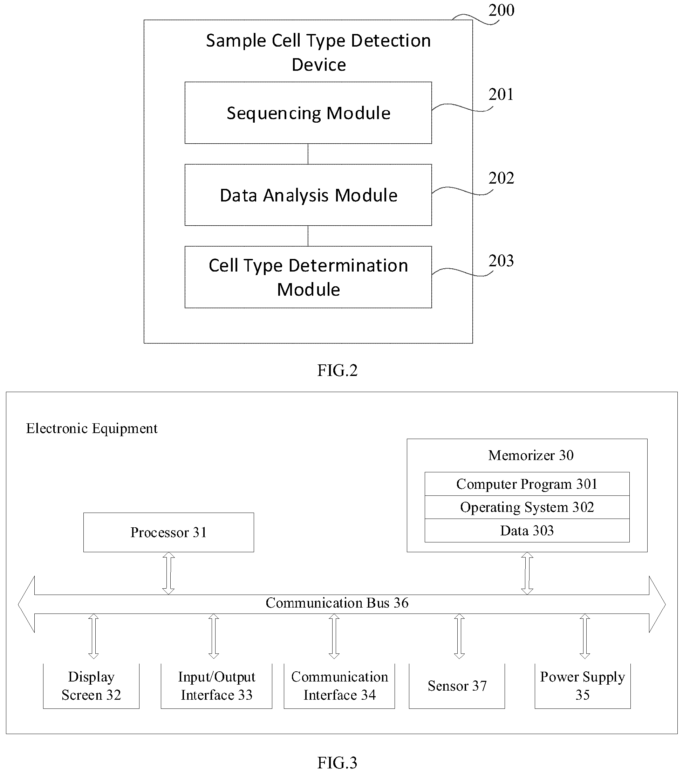

Based on a functional module perspective, see FIG.2, which shows a structural diagram of a biological sample cell composition detection device 200 provided by embodiments of the present invention in a specific embodiment, which may comprise:

Sequencing module 201 for performing single-cell transcriptome sequencing of the biological sample to be detected and obtaining single-cell sequencing results.

A data analysis module 202 for generating a cell gene expression matrix by analyzing single cell sequencing results.

A cell type determination module 203 for determining a cell type contained in a biological sample to be detected by performing a single-cell bioinformatics analysis of a cell gene expression matrix.

Optionally, in some embodiments of the present embodiment, the above device may further comprise:

An annotation module for mapping functional cell gene expression matrix information to a cell gene expression matrix for preliminary cell annotation.

As an optional embodiment of the above embodiment, the above device may further comprise an annotation confirmation module for generating and displaying a map of functional cell target gene expression amounts and cell expression ratios in response to a functional cell target gene expression clustering map drawing instruction; and generating, in response to an annotation confirmation result input instruction, an annotation confirmation message comprising the cell types to which each cell taxon belongs.

As another optional embodiment of the above embodiment, the above device may further comprise a trajectory analysis module for generating gene splice data by analyzing single-cell sequencing results; and based on the gene splice data, performing trajectory analysis on the annotated cell taxa, so as to confirm whether or not the annotated taxa of the cells are fitted to a biological developmental trajectory.

As one other optional embodiment of the above embodiment, the above device may, for example, further comprise a result generation module for counting the cell composition ratios and generating a cell composition ratio detect result of the biological sample to be detected.

Optionally, in other embodiments of the present embodiment, the above device may further comprise a validation module for generating and displaying a map of functional cell target gene expression amounts and cell expression ratios in response to a functional cell target gene expression clustering map drawing instruction; and generating cell type annotation accuracy information for annotated cell taxa in response to an accuracy information input instruction.

Optionally, in some other embodiments of the present embodiment, the above cell type determination module 203 may be further used to: invoke a single-cell transcription data analysis function to perform a single-cell transcription data analysis on a cell gene expression matrix in an interactive computing environment to obtain functional cell gene expression matrix information.

As an optional embodiment of the above embodiment, the above cell type determination module 203 may also be used to: invoke a cell gene calculation relational formula to calculate the number of mitochondrial genes, cell genes, and the total number of gene fragments detected in each cell in a cell gene expression matrix; in response to a filtering parameter setting instruction, invoke a cell filtering relational formula and a gene filtering relational formula, respectively, to filter out the genes and the cells the detection quality of which does not satisfy the predetermined quality conditions, filter out the genes and cells whose detection quality does not meet the preset quality conditions, and obtain the target cell gene data; invoke the cell cycle evaluation relational formula to determine the cell cycle of each cell in the target cell gene data; in response to the data normalization processing instruction, perform data normalization processing on the target cell gene data and perform dimensionality reduction processing on the normalized data; in response to the cell clustering processing instruction, perform cell clustering on the dimensionality reduction processing data to get the cell clustering information.

As another optional embodiment of the above embodiment, the above cell type determination module 203 may further be used to: invoke a normalization relational formula to carry out normalization of a cell gene expression matrix; invoke a logarithmic conversion relational formula to carry out logarithmic conversion of the normalized processed data; and invoke an abnormal gene removal relational formula to remove abnormally high expressed genes in the logarithmically converted data.

Optionally, in some other embodiments of the present embodiment, the above-described device may further comprise a result verification module, which may comprise:

A stability verification unit for generating inter-batch cell composition ratio stability results by analyzing cell composition ratio data for each batch of biological samples; the biological samples to be detected comprise a plurality of batches of biological samples, and the single-cell sequencing results comprise a plurality of sets of single-cell sequencing results carrying batch information.

A change verification unit for obtaining, for a biological sample at each time point, cell composition ratio data determined for the biological sample at the current time; and generating cell composition ratio change information by performing a time series analysis of the cell composition ratio data of the biological sample at different times. The biological sample to be detected is sampled at multiple time points of the same biological sample, and the single-cell sequencing results include single-cell sequencing results at multiple time points of the same biological sample.

The functions of each functional module of the biological sample cell composition ratio detection device described in the embodiment of the present invention may be specifically realized in accordance with the method in the above method embodiment, and the specific realization process thereof may be referred to the relevant descriptions of the above method embodiment and will not be repeated herein.

As can be seen from the above, embodiments of the present invention enable low-cost, high-throughput, accurate and quantitative one-step detection of the cell composition of a biological sample.

The biological sample cell composition detection device mentioned above is described from the perspective of a functional module, and further, the present application also provides an electronic device, which is described from the perspective of hardware. FIG.3 shows a schematic diagram of the structure of the electronic device provided by an embodiment of the present application in one embodiment. As shown in FIG.3, the electronic device comprises a memory 30 for storing a computer program; and a processor 31 for implementing steps of the biological sample cell composition detection method as mentioned in any of the above embodiments when executing the computer program.

Wherein, the processor 31 may include one or more processing cores, such as a 4-core processor, an 8-core processor, and the processor 31 may also be a controller, a microcontroller, a microprocessor, or other data processing chip, etc. The processor 31 may employ a DSP (Digital Signal Processing), an FPGA (Field-Programmable Gate Array), a PLA (Programmable Logic Array), a programmable Logic Array) to be implemented in at least one form of hardware. The processor 31 may also include a main processor and a co-processor, the main processor being a processor, also referred to as a CPU (Central Processing Unit, Central Processing Unit), for processing data in a wake-up state; and the co-processor being a low-power processor for processing data in a standby state. In some embodiments, the processor 31 may be integrated with a GPU (Graphics Processing Unit), which is used to be responsible for rendering and drawing the content to be displayed by the display. In some embodiments, the processor 31 may also include an AI (Artificial Intelligence) processor that is used to handle computational operations related to machine learning.

The memory 30 may include one or more computer-readable storage media, which may be non-transitory. The memory 30 may also include high-speed random-access memory as well as non-volatile memory, such as one or more disk storage devices, flash memory storage devices. The memory 30 may in some embodiments be an internal storage unit of an electronic device, such as a hard drive of a server. The memory 30 may also be, in other embodiments, an external storage device of an electronic device, such as a plug-in hard drive equipped on a server, a Smart Media Card (SMC), a Secure Digital (SD) card, a Flash Card, and the like. Further, the memory 30 may also include both an internal storage unit of the electronic device and an external storage device. The memory 30 may be used not only for storing application software and various types of data installed in the electronic device, e.g., code for executing a program during a method for detecting the cell composition of a biological sample, etc., but may also be used for temporarily storing data that has been or will be output. In this embodiment, memory 30 is used to store at least the following computer program 301, wherein the computer program, after being loaded and executed by the processor 31, can realize the relevant steps of the method of detecting the cell composition of a biological sample disclosed in any of the preceding embodiments. In addition, the resources stored in the memory 30 may also include an operating system 302 and data 303, etc., and the storage method may be transient storage or permanent storage. Wherein, the operating system 302 may include Windows, Unix, Linux, and the like. The data 303 may include, but is not limited to, data corresponding to the cell composition detection results of the biological sample, and the like.

In some embodiments, the above electronic device may also include a display 32, an input/output interface 33, a communication interface 34 or called a network interface, a power supply 35, and a communication bus 36, wherein the display 32, the input/output interface 33, such as a keyboard, belong to a user interface, and optional user interfaces may include a standard wired interface, a wireless interface, and the like. Optionally, in some embodiments, the display may be an LED display, an LCD display, a touchscreen LCD display, and an OLED (Organic Light-Emitting Diode) touchscreen, and the like. The display may also be appropriately referred to as a display or display unit for displaying information processed in the electronic device and for displaying a visual user interface. The communication interface 34 optionally may include a wired interface and/or a wireless interface, such as a WI-FI interface, a Bluetooth interface, etc., and is typically used to establish a communication connection between the electronic device and other electronic devices. The communication bus 36 may be a peripheral component interconnect standard (peripheral component interconnect, or PCI) bus or an extended industry standard architecture (extended industry standard architecture, or EISA) bus, among others. The bus can be divided into address bus, data bus, control bus and so on. For ease of representation, FIG.3 is represented by only one thick line, but does not indicate that there is only one bus or one type of bus.

It will be appreciated by those skilled in the art that the structure illustrated in FIG.3 does not constitute a limitation of the electronic device, and may include more or fewer components than illustrated, such as sensors 37 that implement various types of functions may also be included.

The functions of each functional module of the electronic device described in the embodiment of the present invention may be specifically realized according to the method in the above method embodiment, and the specific realization process thereof may be referred to the relevant description of the above method embodiment and will not be repeated herein.

As can be seen from the above, embodiments of the present invention enable low-cost, high-throughput, accurate and quantitative one-step detection of the cell composition of a biological sample.

It is to be understood that the method of detecting the cell composition of a biological sample in the above embodiment may be stored in a computer readable storage medium if it is implemented in the form of a software functional unit and sold or used as a stand-alone product. Based on this understanding, the technical solution of the present application in essence or the part that contributes to the prior art or all or part of the technical solution may be embodied in the form of a software product that is stored in a storage medium that performs all or part of the steps of the method of the various embodiments of the present application. And the storage media include USB flash drives, removable hard drives, Read-Only Memory (ROM), Random Access Memory (RAM), electrically erasable programmable ROM, registers, hard disks, multimedia cards, card-type memories (e.g., SD or DX memories, etc.), magnetic memories, removable Disks, CD-ROMs, disks or CD-ROMs, and other media on which program code can be stored.

Based on this, embodiments of the present invention also provide a readable storage medium storing a computer program, said computer program being executed by a processor as in the steps of the method of detecting cell composition of a biological sample described in any of the above embodiments.

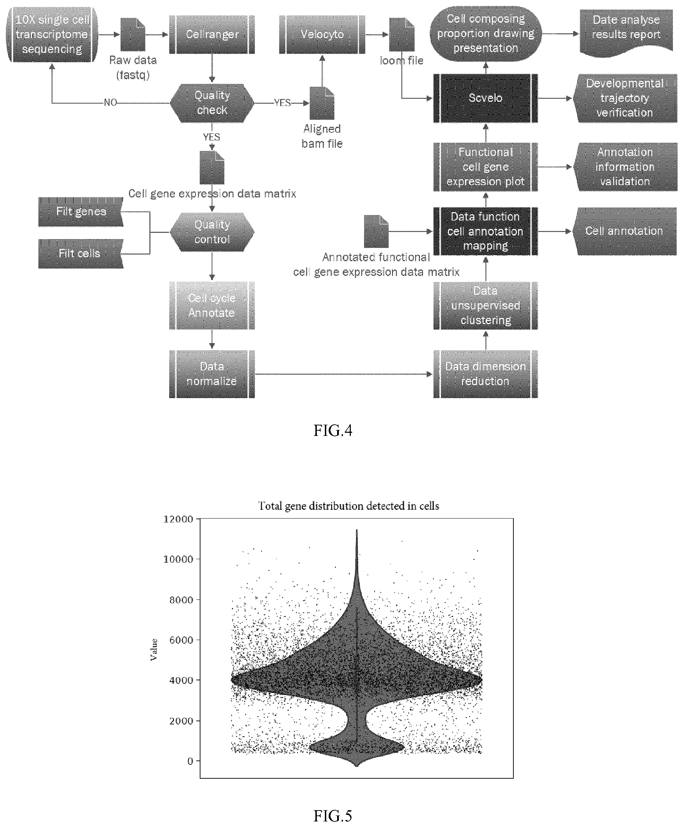

Finally, in order to make the technical solutions of the present application more clearly understood by those skilled in the field to which it belongs, the present application also gives a schematic example in connection with FIG.4, which may include the following:

One million cell samples or tissues are obtained from the biological samples to be detected and dissociated into single cells, and the NPC cells are sequenced at a depth of 150G using the 10X GENOMICS sequencing platform, and the single-cell detect results are obtained. The single cell sequencing results are subjected to bioinformatic analysis, and the bioinformatic analysis step may include the following:

A1: Obtain the downstream format data of 10X GENOMICS sequencing platform, i.e. fastq sequence data, and use the mkref function of Cell ranger software,

i.e. the reference sequence alignment database construction function, to reference the human GRCh38 (a version number of the human reference genome) version of the genome, GENECODE (a database of gene annotation information, is also known as the Gene Annotation Information Database), the latest version of the database annotation files constructed comparison indexes. Single-cell sequencing data expression matrices were obtained using the Cell ranger software count function and its default parameter analysis. The output data of Cell ranger software, i.e. the expression matrix of single-cell sequencing data, was quality checked, and if it passed the quality check, the cell gene expression matrix was generated, and the bam file for sequence comparison was generated at the same time. If the quality check is not passed, the 10X GENOMICS sequencing platform is used to re-sequence the single cell transcriptome of the biological sample to be detected. Optionally, this can be achieved, for example, by the following computer program:

cellranger count --id=hNPC --fastqs=DATA_DIR --transcriptome=GRCh38- Genecode --localcores=16 --localmem=80.

A2: Processing of bam files generated by Cell ranger analysis, i.e. analyzing cell gene splice rates, using the run10x function of the velocyto software, to obtain a cell gene splice rate loom file, a loom file is generated by the velocyto software for storing the number of spliced/unspliced transcripts, which is subsequently used to analyze cell development trajectories. Optionally, this can be achieved, for example, by the following computer program:

velocyto run10x -m human38_rmsk.gtf DATA_DIR genes.gtf

A3: The functions in the following steps can be pre-compiled on Python to facilitate timely adjustment of optimization parameters, or of course, they can be embedded in the background and imported. After pre-compiling the functions in the following steps, single-cell transcriptome data were analyzed using Scanpy on the Python platform:

A31: Use the function scvelo.read to read a loom file generated by the velocyto software, which can be represented, for example, as velo.loom. optionally, this can be realized, for example, by the following computer program:

ldata = scvelo.read(velo.loom, cache=True)

A32: Use the function read_cellranger to read the filtered cell gene expression matrix file analyzed and processed by the Cell ranger software, which can be represented, for example, as filtered_feature_bc_matrix.h5. Optionally, this can be achieved, for example, by the following computer program:

count_data=read_Cellranger(filtered_feature_bc_matrix.h5, regex_only=True, add_sample_id=False, cache=-False)

A33: The cell mitochondrial genes and ribosomal genes are identified and their percentage of detection in the cell is calculated using the function annotate_qc_metrics. Optionally, this can be achieved, for example, by the following computer program:

count_data = annotate_qc_metrics(count_data)

A34: Use the functions filtering_cell and filtering_gene to set specific filtering parameters to filter the data for quality control, in which the filtering parameters can be modified according to the data situation. It is also possible to use filtering_cell and filtering_gene to adjust the filtering parameters according to the data situation to filter out genes and cells with poor detection quality. Optionally, this may be achieved, for example, by the following computer program:

count_data=filtering_cell(count_data, min_t_count=1500, max_t_count=150,000, min_t_gene=1500, max_t_-gene=10,000, min_mito_percent =0, max_mito_percent=0.5)

count_data=filtering_gene(count_data, min_cells=10)

A35: Confirmation of the evaluation of the cell cycle using the function annotate_cellcycle is optionally possible, e.g. by the following computer program:
annotate_cellcycle( count_data )
A36: The cell gene expression matrix is normalized using the scanpy.pp.normalize_total function, and the scanpy.pp.log1p function performs a logarithmic transformation of the normalized data, and then the above data is calculated and analyzed by the scanpy .pp.recipe_zheng17 function to remove abnormally high expressed genes. Optionally, this may be achieved, for example, by the following computer program:

scanpy.pp.normalise_total( count_data , exclude_highly_expressed= True, max_fraction=0.05, inplace=True)

scanpy.pp.loglp(count_data) scanpy.pp.recipe_zheng17(count_data, n_top_genes=3000, log= False, plot=False, copy=False)

A37: The data obtained from the above step is subjected to principal component analysis using the scanpy.tl.pca function, and the data obtained from the above step is subjected to dimensionality reduction using the UMAP function or the tSNE function. Optionally, this may be achieved, for example, by the following computer program:

```
scanpy.tl.pca(count_data)
scanpy.pl.pca_overview( count_data ) cpm_nml_umap=umap.UMAP(n_neighbors=30, min_dist=0.9, n_components=2, random_state=42).fit_-
transform(count_data.obsm["X_pca "])
```

A38: Unsupervised clustering of the data obtained in the above steps is performed using the scanpy.pp.neighbour function or the Leiden or Louvain algorithms, wherein the resolution may be adjusted according to the clustering. Optionally, this may be achieved, for example, by the following computer program:

```
scanpy.pp.neighbors(count_data, n_neighbors=50, n_pcs=30, use_rep="X_pca", knn=True, random_state=0,
method='umap', metric='euclidean')
scanpy.tl.leiden(count_data, resolution=2, key_added="leiden2")
```

A4: Use scGCN software to map functional cell gene expression matrix information to assay data such as filtered_feature_bc_matrix.hd5 data for rapid identification of cell types and statistics on the proportion of each type of cell composition.

A5: Plot expression of functional cell target genes and proportion of cell expression to confirm cell type annotation accuracy.

A6: Trajectory analysis of the annotated cell taxa using the scvelo software, confirming whether the annotation information fits the biological developmental trajectory. Optionally, this can be achieved, for example, by the following computer program:

```
adata_combine = scv.utils.merge(count_data, ldata)
```

```
scvelo.pp.filter_and_normalise(adata_combine, min_shared_counts= 10, n_top_genes=5000)
```

```
scvelo.pp.moments(adata_combine, n_pcs=30, n_neighbors=30) scvelo.tl.recover_dynamics(adata_combine,
n_jobs=36)
```

```
scvelo.tl.velocity(adata_combine, mode='dynamic', n_jobs=36) scvelo.tl.velocity_graph(adata_combine,
n_jobs=36)
```

```
scvelo.pl.velocity_embedding_stream(adata_combine, basis='umap', colour='cell_type_annot2',X=adata_combine.obsm["X_umap"])
```

A7: Calculating the proportions of each cell type and plotting a pie chart of the distribution of cell types and a bar chart of the cell cycle of each cell type using the self-written functions cell_proportion_pieplot and cell_proportion_barplot. Optionally, this can be achieved, for example, by the following computer program:

```
cell_proportion_pieplot(count_data, x="cell_type_annot2", save="Cell_tp_prpie.pdf")
```

```
cell_proportion_barplot(count_data, x="cell_type_annot2", y="phase", colour=colors_leiden_2, save =
"Cell_tp_prbar.pdf")
```

A8: Finally, one clicks to generate a pdf version analysis report without code.

In this step, can be pre-installed in the analysis of the server pandoc, TeXLive and nbextensions, in the jupyter analysis of the file menu bar File inside the Download as the choice of PDF via LaTeX (.pdf), you can automatically generate the analysis of the current file report.

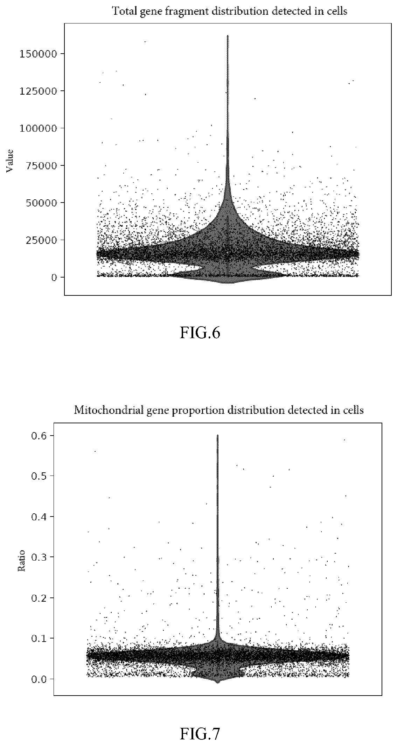

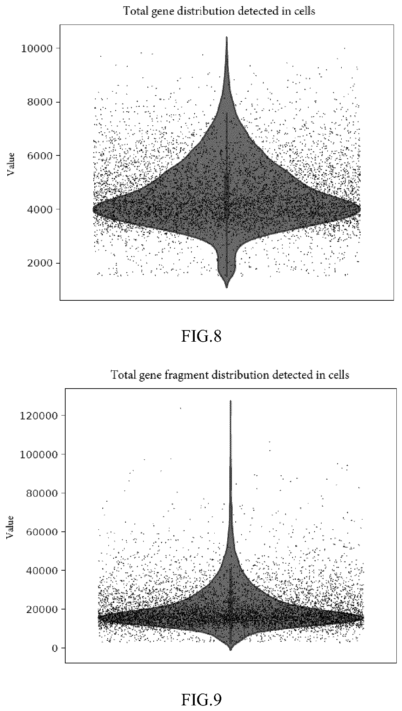

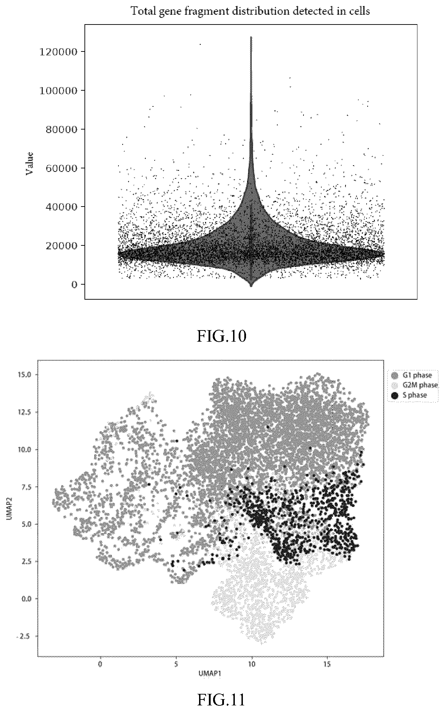

In order to verify the technical solutions provided in this embodiment, the present application was processed accordingly based on specific samples in accordance with the method described above, as shown in FIG.5- FIG.23, FIG.5 is a violin plot of the total number of genes detected in the sample cells before filtration, wherein the vertical coordinate indicates the total number of genes detected; FIG.6 is a violin plot of the total number of genes fragmented before filtration in the sample cells, wherein the vertical coordinate indicates the total number of genes fragmented; FIG.7 is a violin plot of the total number of genes detected in the sample mitochondrial gene detection proportion before cell filtration violin plot, where the vertical coordinate indicates the proportion of cell mitochondrial gene detection; FIG.8 shows the total number of gene detection after sample cell filtration violin plot, where the vertical coordinate indicates the total number of gene detection; FIG.9 shows the total number of gene fragment detection after sample cell filtration violin plot, where the vertical coordinate indicates the total number of gene fragment detection; FIG.10 shows the total number of mitochondrial gene detection after sample cell filtration Proportion violin plot, where the vertical coordinate indicates the proportion of cell mitochondrial gene detection;

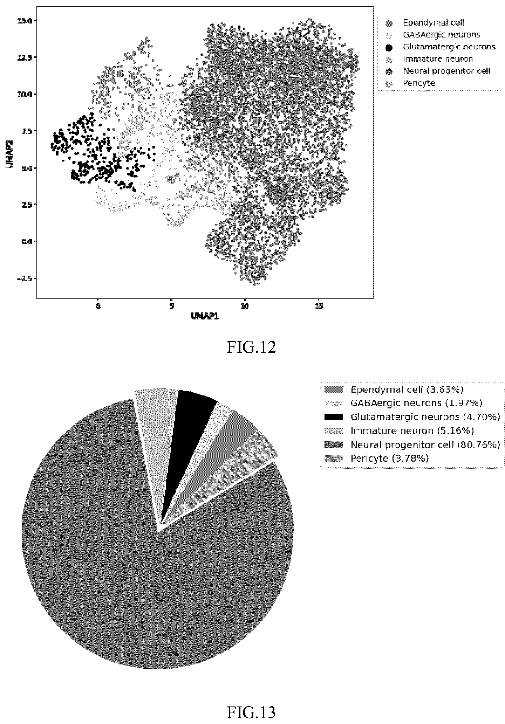

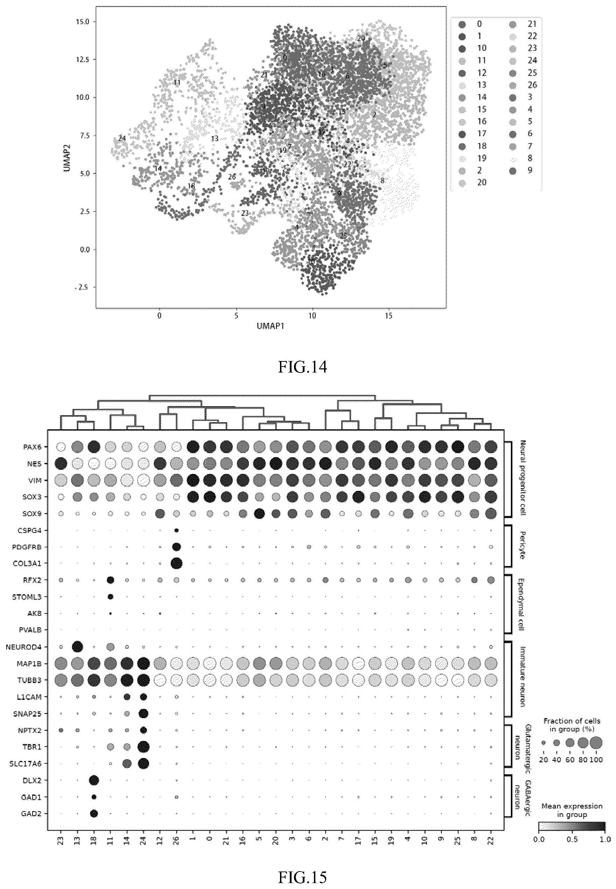

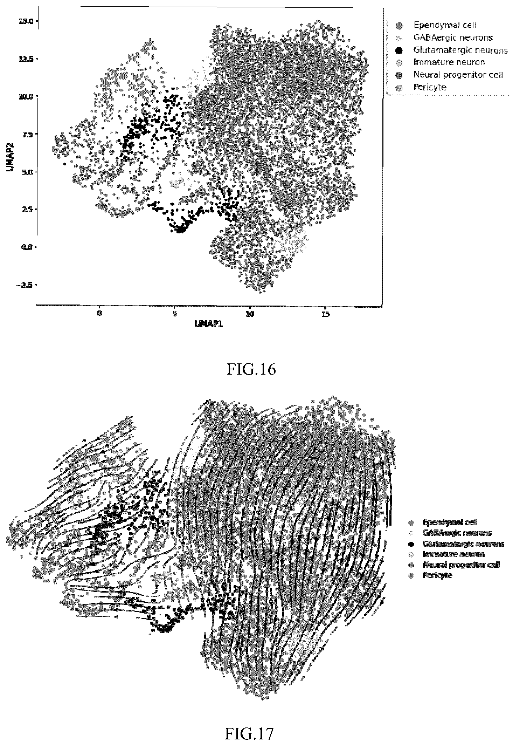

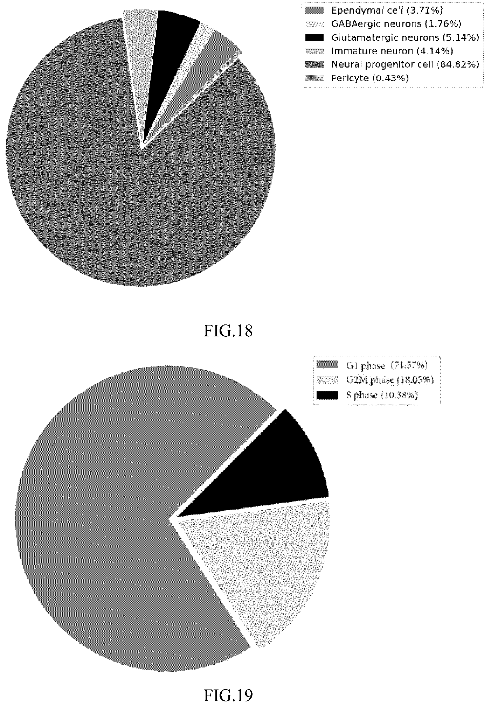

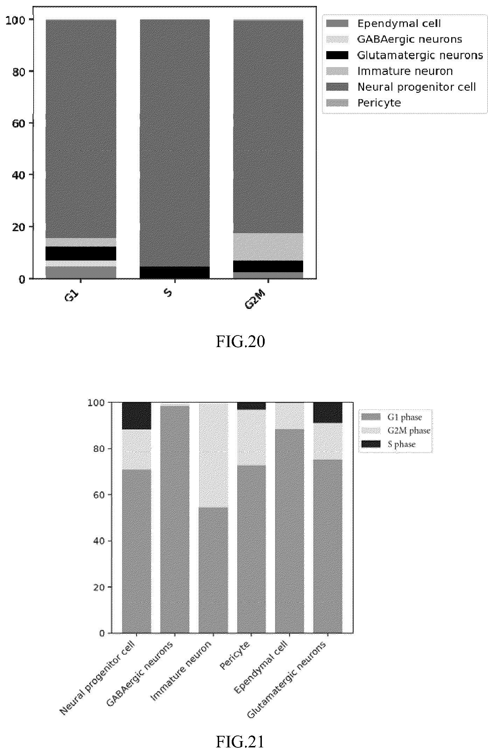

FIG.11 shows the data degradation cell cycle distribution map, where the horizontal coordinate indicates dimension 1 and the vertical coordinate indicates dimension 2; FIG.12 shows the data degradation functional cell matrix mapping cell annotation map, where the horizontal coordinate indicates dimension 1 and the vertical coordinate indicates dimension 2; FIG.13 shows the proportionality of the composition of the functional cell matrix mapping annotated cells pie chart; FIG.14 shows the data degradation cell clustering map, where the horizontal coordinate indicates dimension 1 and the vertical coordinate denotes dimension 2; FIG.15 is a cell clustering target gene expression bubble map, where the horizontal coordinate denotes the cell class group and the vertical coordinate denotes the gene; FIG.16 is a data downscaling target gene expression correction cell annotation map, where the horizontal coordinate denotes dimension 1 and the vertical coordinate denotes dimension 2; FIG.17 is a data downscaling cell trajectory map; FIG.18 is a pie chart of the proportionality of the composition of the annotations of cells for confirmation of biological developmental trajectories; FIG.19 is a pie chart of cell cycle proportions; FIG.20 is a bar chart of cell cycle composition proportions, where the horizontal coordinates represent cell cycles and the vertical coordinates represent cell proportions; and FIG.21 is a bar chart of cell composition cycle proportions, where the horizontal coordinates represent cell types and the vertical coordinates represent cell proportions.

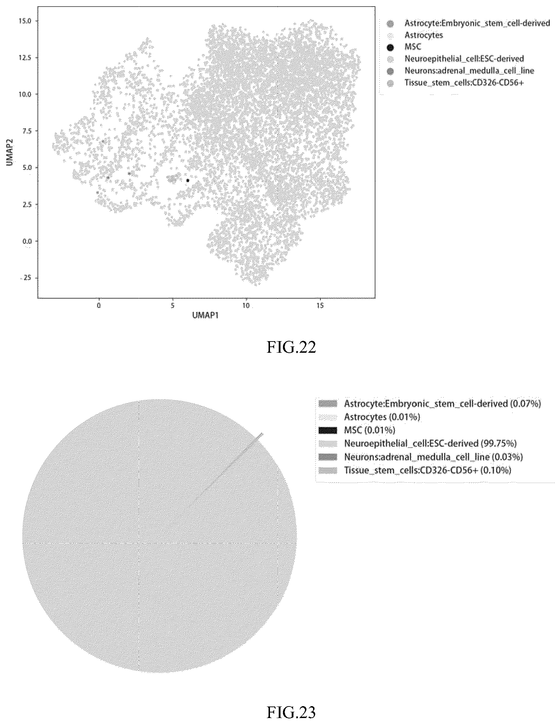

Meanwhile, the single-cell transcriptome sequencing data of the present embodiment was rapidly annotated using the SingleR software based multi-cell annotation database (Human Primary Cell Atlas Data and Blueprint Encode Data) method as shown in FIG.22- FIG.23, where FIG.22 shows the data downscaling based on the SingleR software multi-cell FIG.22 shows the cell annotation diagram of the SingleR software-based multicell annotation database, where the horizontal coordinate indicates dimension 1 and the vertical coordinate indicates dimension 2; FIG.23 shows the pie chart of the proportion of cell annotation composition of the SingleR software-based multicell annotation database. The results show that the information annotated by this method is basically inaccurate, and the sample of the embodiment is neural progenitor cells and their differentiated neurons, but the SingleR annotation results do not contain neural progenitor cells at all, and even appear the annotated taxa of other tissue and organ cells. And the invention can quickly and accurately annotate the real cell type, as FIG.12-FIG.13 presents the results; and through the multi-target gene verification to enhance the accuracy of the annotation, as FIG.15-FIG.16 presents the results, confirmed by the multi-target genes, especially the pericyte annotation ratio annotation is more accurate, correcting the rapid mapping annotation bias; and finally the cell trajectory is confirmed, as FIG.17 presents the results, the neuronal differentiation of neurons from neural progenitor cells to immature Finally, the cell trajectory was confirmed, as shown in FIG.17, from neural progenitor cells to immature neurons and then to terminally differentiated cells (pericytes, ventricular cells, glutamatergic neurons and γ-aminobutyric acid neurons), which was confirmed to be correctly annotated by fitting with the biological developmental trajectory.

As can be seen from the above, the embodiments of the present invention have high throughput, high accuracy, high resolution and high reproducibility, and can achieve low-cost, high-throughput, accurate and quantitative one-step detection of the cell composition of biological samples.

Each embodiment in this specification is described in a progressive manner, and each embodiment focuses on the differences with other embodiments, and each embodiment is the same or similar to each other, see the same or similar parts of each embodiment. For the hardware disclosed in the embodiments, including devices and electronic equipment, since it corresponds to the methods disclosed in the embodiments, the description is relatively simple, and the relevant parts can be found in the method section.

The professional may further realize that the units and algorithmic steps of the various examples described in conjunction with the embodiments disclosed herein are capable of being implemented in electronic hardware, computer software, or a combination of both, and that in order to clearly illustrate the interchangeability of the hardware and the software, the compositions and the steps of the various examples have been described in the foregoing description in general terms according to function. Whether these functions are performed in hardware or software depends on the particular application and design constraints of the technical solution. The skilled person may use different methods to implement the described functions for each particular application, but such implementations should not be considered outside the scope of the present invention.

The above describes in detail a biological sample cell composition detection method, apparatus, electronic device and readable storage medium provided in this application. Specific examples have been applied herein to illustrate the principles and embodiments of the present invention, and the above illustrations of the embodiments are only used to help understand the method of the present invention and its core ideas. It should be noted that, for the person of ordinary skill in the art, without departing from the principle of the present invention, several improvements and modifications can be made to the present application, which also fall within the scope of protection of the claims of the present application.

**Claims**

1.  A method of detecting cell composition of a biological sample, wherein:

   single-cell transcriptome sequencing is performed on the biological sample to be detected to obtain a single-cell sequencing result;

a cell gene expression matrix is generated by analyzing the single-cell sequencing result; and

cell types comprised in the biological sample to be detected are determined by performing single-cell bioinformatics analysis on the cell gene expression matrix.

2. The method of detecting cell composition of a biological sample according to claim 1, wherein, after performing single-cell bioinformatics analysis on the cell gene expression matrix, the method further comprises:

information of a functional cell gene expression matrix is mapped to the cell gene expression matrix to perform preliminary cell annotation.

3. The method of detecting cell composition of a biological sample according to claim 2, wherein, after mapping the information of the functional cell gene expression matrix to the cell gene expression matrix, the method further comprises:

in response to a command to plot a cluster diagram of functional cell target gene expression, a diagram showing functional cell target gene expression levels and cell expression proportions is generated and displayed;

in response to a command to input annotation confirmation result, annotation confirmation information comprising cell types to which each cell cluster belongs are generated.

4. The method of detecting cell composition of a biological sample according to any one of claims 1 to 3, wherein, after performing the single-cell bioinformatic analysis on the cell gene expression matrix, the method further comprises:

gene splicing data are generated by analyzing the single-cell sequencing results;

trajectory analysis is performed on annotated cell clusters based on the gene splicing data to confirm whether the annotated cell clusters fit biological development trajectories.

5. The method of detecting cell composition of a biological sample according to any one of claims 1 to 4, wherein, after determining the cell types comprised in the biological sample to be detected, the method further comprises:

cell composition proportions are calculated, and cell composition proportion detection results for the biological sample to be detected are generated.

6. The method of detecting cell composition of a biological sample according to any one of claims 1 to 5, wherein, the cell types comprised in the biological sample to be detected being determined by performing single-cell bioinformatics analysis of the cell gene expression matrix, comprising:

single-cell transcriptional data analysis is performed on the cell gene expression matrix in an interactive computing environment to obtain the cell types comprised in the biological sample to be detected.

7. The method of detecting cell composition of a biological sample according to claim 6, wherein the cell types comprised in the biological sample to be detected are determined, by performing single-cell bioinformatics analysis on the cell gene expression matrix, comprising:

a cell gene calculation relational formula is invoked to calculate the proportion of cell mitochondrial genes, the total number of genes detected in the cells, the total number of gene fragments detected in the cells and the total number of fragments detected in the gene and the total number of cells detected in the cell gene expression matrix;

in response to the filtering parameter setting command, the cell filtering relational formula and

the gene filtering relational formula are invoked respectively to filter out the genes and cells whose quality detected does not satisfy the preset quality conditions, and to obtain the target cell gene data;

in response to the data normalization processing command, data normalization processing is performed on the target cell gene data, and dimensionality reduction processing is performed on the normalized data;

in response to the cell clustering processing command, cell clustering is performed on the dimensionality reduction processed data to obtain the cell subcluster information.

8. The method of detecting cell composition of a biological sample according to claim 7, wherein, after obtaining the target cell genetic data, the method further comprises:

a cell cycle assessment relational formula is invoked to determine the cell cycle of each cell in the target cell genetic data.

9. The method of detecting cell composition of a biological sample according to claim 7, wherein in response to data normalization processing command, the data normalization is performed on the cell gene expression matrix, comprising:

a normalization relational formula is invoked to normalize the cell gene expression matrix;
a logarithmic conversion relational formula is invoked to perform logarithmic conversion on normalized data;
an abnormal gene removal relational formula is invoked to remove abnormally high expressed genes in the log-transformed data.

10. The method of detecting cell composition of a biological sample according to any one of claims 1 to 5, wherein the biological sample to be detected comprises a plurality of batches of biological samples, the single-cell sequencing results comprise a plurality of sets of single-cell sequencing results carrying information about the batches; and after determining the cell types comprised in the biological samples to be detected, the method further comprises:
by analyzing cell composition proportion data for each batch of biological samples, inter-batch cell composition proportion stability results are generated.

11. The method of detecting cell composition of a biological sample according to any one of claims 1 to 5, wherein the biological sample to be detected is sampled from the same biological sample at a plurality of time points, the single-cell sequencing results comprise single-cell sequencing results from the same biological sample at a plurality of time points; and after determining the cell types comprised in the biological sample to be detected, the method further comprises:

for biological sample of each time point, cell composition proportion data of the biological sample at the current time is obtained;
by time-series analysis of cell composition proportion data of the biological sample at different times, information on changes of cell composition proportions is generated.

12. A device for detecting biological sample cell composition, comprising:

a sequencing module used for performing single-cell transcriptome sequencing on the biological sample to be detected and obtaining single-cell sequencing results;
a data analysis module used for generating cell gene expression matrix by analyzing the single-cell sequencing results;
a cell type determination module used for determining cell types comprised in the biological sample to be detected by performing single-cell bioinformatics analysis on the cell gene expression matrix.

13. An electronic device, comprising a processor and a memory, wherein the processor is used to implement the steps of the method of detecting cell composition of a biological sample of any one of claims 1 to 11 when executing a computer program stored in the memory.

14. A readable storage medium wherein the readable storage medium stores a computer program, and the computer program is executed by a processor to implement the steps of the method of detecting cell composition of a biological sample of any one of claims 1 to 11.

| Perform single-cell transcriptome sequencing on the sample to be detected to obtain single-cell sequencing results. | S101 |

| Analyze the single-cell sequencing results to generate a cell gene expression matrix. | S102 |

| Conduct single-cell bioinformatics analysis on the cell gene expression matrix to determine the cell types contained in the biological sample to be detected. | S103 |

FIG.1

200

Sample Cell Type Detection
Device

Sequencing Module 201

Data Analysis Module 202

Cell Type Determination
Module 203

FIG.2

Electronic Equipment

Memorizer 30

Computer Program 301

Operating System 302

Data 303

Processor 31

Communication Bus 36

Display
Screen 32

Input/Output
Interface 33

Communication
Interface 34

Sensor 37

Power Supply
35

FIG.3

FIG.4

FIG.5

Total gene fragment distribution detected in cells

FIG.6

Mitochondrial gene proportion distribution detected in cells

FIG.7

Total gene distribution detected in cells

FIG.8

Total gene fragment distribution detected in cells

FIG.9

Total gene fragment distribution detected in cells

FIG.10

FIG.11

FIG.12

FIG.13

FIG.14

FIG.15

FIG.16

FIG.17

Ependymal cell (3.71%)
GABAergic neurons (1.76%)
Glutamatergic neurons (5.14%)
Immature neuron (4.14%)
Neural progenitor cell (84.82%)
Pericyte (0.43%)

FIG.18

G1 phase (71.57%)
G2M phase (18.05%)
S phase (10.38%)

FIG.19

FIG.20

FIG.21

FIG.22

FIG.23

## INTERNATIONAL SEARCH REPORT

| International application No. |
| --- |
| **PCT/CN2023/113130** |

### A. CLASSIFICATION OF SUBJECT MATTER

G16B20/00(2019.01)i

According to International Patent Classification (IPC) or to both national classification and IPC

### B. FIELDS SEARCHED

Minimum documentation searched (classification system followed by classification symbols)

IPC:G16B

Documentation searched other than minimum documentation to the extent that such documents are included in the fields searched

Electronic data base consulted during the international search (name of data base and, where practicable, search terms used)

CNTXT; CNABS; VEN; USTXT; EPTXT; WOTXT; CNKI; IEEE: 样本, 单个, 细胞, 转录, 测序, 基因, 表达矩阵, 类型, 类别, 分类, 聚类, 注释, sample, single, cell, transcription, sequence, gene, expression matrix, type, category, classification, cluster, note

### C. DOCUMENTS CONSIDERED TO BE RELEVANT

| Category* | Citation of document, with indication, where appropriate, of the relevant passages | Relevant to claim No. |
| --- | --- | --- |
| X | CN 111951892 A (UEC GROUP LTD.) 17 November 2020 (2020-11-17) description, paragraphs 47-109 | 1-14 |
| X | CN 109979538 A (GUANGZHOU GENE DENOVO BIOTECHNOLOGY CO., LTD.) 05 July 2019 (2019-07-05) description, paragraphs 67-113 | 1-14 |
| X | CN 112270953 A (HARBIN YINJI TECHNOLOGY CO., LTD.) 26 January 2021 (2021-01-26) description, paragraphs 42-109 | 1-14 |
| X | CN 113963747 A (NOVEL BIOTECHNOLOGY INC.) 21 January 2022 (2022-01-21) description, paragraphs 16-23 | 1-14 |
| A | US 2022068438 A1 (THE BROAD INSTITUTE INC. et al.) 03 March 2022 (2022-03-03) entire document | 1-14 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **31 October 2023** | **03 November 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

| Patent document cited in search report | | | Publication date (day/month/year) | Patent family member(s) | | | Publication date (day/month/year) |
|---|---|---|---|---|---|---|---|
| CN | 111951892 | A | 17 November 2020 | None | | | |
| CN | 109979538 | A | 05 July 2019 | CN | 109979538 | B | 01 October 2021 |
| CN | 112270953 | A | 26 January 2021 | None | | | |
| CN | 113963747 | A | 21 January 2022 | None | | | |
| US | 2022068438 | A1 | 03 March 2022 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023113130 W
- CN 202210982600